# EUROPEAN PATENT APPLICATION

(11) **EP 1 285 965 A1**
(43) Date of publication of application: **26.02.2003**
(21) Application number: 01118967.7
(22) Date of filing: 06.08.2001
(51) Int. Cl.: C12N 15/29, C12N 15/82, C12N 5/10, C07K 14/415, A01H 5/00

(54) **Bifactorial endosperm box, trans-activating factor that binds thereto, and method of regulation of promoter activity**

(71) Applicant: MERISTEM THERAPEUTICS, 63100 Clermont-Ferrand (FR)
(72) Inventor: Norre, Frédéric, 63730 Mirefleurs (FR); Gruber, Véronique, Dr., 63540 Romagnat (FR)
(74) Representative: Richebourg, Michel François

(57) **Abstract**

The present invention concerns a bifactorial endosperm box, a *trans*-activating factor that binds thereto and methods for regulating promoter activity.

## Description

The present invention relates generally to plant promoters, and more particularly to the regulation of the activity of said promoters, and the way such regulation can be used to increase expression of endogenous or heterologous proteins in plants containing such regulated promoters, for example to increase production of agricultural, industrial, therapeutic, cosmetic, prophylactic or nutritional proteins.

Many plant promoters have been discovered and isolated over the years of plant biotechnology research and development, and have been used in recombinant plant expression systems to attempt to increase the expression of proteins or polypeptides which are of importance to the agricultural, chemical, agri-food, cosmetic and pharmaceutical industries, such as proteins, enzymes, and therapeutic molecules. However, one of the problems that still remains unsolved in a satisfying manner is that of the fine control of the regulation of promoter activity during the plant's development, either in order to reduce expression of a particular protein, polypeptide, metabolite, or to increase it.

Research into the understanding of the activity of such plant promoters has been oriented towards the discovery and isolation of transcription factors, since these factors have been indicated as being directly involved in the regulation of the activity of the promoter through binding to certain nucleic acid sequences present on the promoter DNA.

The present applicants have already carried out work on a series of chimeric and synthetic promoters derived from the wheat high molecular weight glutenin promoter (HMWG), as published in PCT application WO 01/23593. This work has led the applicant to the surprising discovery that a region of the chimeric and synthetic promoters that were created was found to be involved in the regulation of the activity of the promoter. The region concerned has been designated by the applicants as an atypical bifactorial endosperm box.

The following terms and expressions used in the description and claims have the following meaning:
- "bifactorial endosperm box" means a natural, synthetic or chimeric nucleic acid sequence that comprises two moieties that bind selectively to one or more *trans-*activating factors and was originally isolated from maize endosperm;
- "*trans*-a*c*tivating factor" means a protein, peptide or sequence of contiguous amino acids that binds specifically to a *cis*-acting element on a nucleic acid sequence and is implicated in the regulation of the functional activity of said nucleic acid sequence;
- "TGA1a-like bZip" trans-activating factor means a protein which is able to bind to the as-1 *cis*-activating motif;
- "O2 trans-activating factor" means an Opaque-2 protein which binds to the opaque 2 *cis*-activating motif;
- "O2 motif" means a nucleic acid sequence having the consensus sequence "TCCACGTAGA";
- "PBF" means a prolamin-box binding factor, belonging to the DOF (DNA binding with One Finger) family of proteins;
- "as-1 motif" means a nucleic acid sequence having the consensus sequence "ACTGACGTAAGGGATGACGCAC";
- "G-box-like motif means a nucleic acid sequence having the consensus sequence "TTACGTGG";
- "ACGT-like zein motif' means a nucleic acid sequence having the consensus sequence "GCACGTCA"
- "prolamin box" or "Pb1 box" means a nucleic acid sequence having the consensus sequence "TG(T/C/A)AAA(G/A)"
- "plant seed specific promoter" means promoter which is capable of expressing nucleic acid sequences which are linked in a functional manner to said promoter, during seed development in a host plant;
- "plant constitutive promoter" means a promoter which is capable of expressing nucleic acid sequences which are linked in a functional manner to said promoter, in all or practically all the tissues of the host plant throughout the development of said plant;
- "plant host" means a whole plant, part thereof, plant cell, plant cellular organ such as a chloroplast or mitochondria, propagule, seed, root, stem, leaf or tuber;
- "molecule of interest" means a molecule that is expressed by the plant host cell that is of industrial interest in the broadest sense, i.e. is applicable to agriculture, chemical industry, pharmaceutical industry, cosmetics industry, and the diagnostics industry for example.

Accordingly, one object of the present invention is a bifactorial endosperm box comprising a 5' upstream G-box-like motif and a 3' downstream prolamine box-like motif.

In a preferred embodiment, the bifactorial endosperm box originates from a wheat high molecular weight glutenin (HMWG) promoter.

In yet another more preferred embodiment, the bifactorial endosperm box originates from the Dx5 HMWG promoter.

In still yet an even more preferred embodiment, the bifactorial endosperm box consists of the nucleic acid sequence identified in the sequence listing as SEQ.ID01.

Another object of the present invention is a method of regulating the activity of a promoter in a plant, wherein the promoter contains a bifactorial endosperm box as described, and said box binds to one or more trans-activating factors produced in the plant.

Still yet another object of the present invention is a method of increasing the expression of at least one molecule of interest in a recombinant plant host cell, comprising introducing into said plant host cell a promoter, comprising the bifactorial endosperm box as described previously, upstream of a nucleic acid sequence coding for at least one molecule of interest, wherein the bifactorial endosperm box binds to one or more trans-activating factors produced in the plant, thereby increasing expression of the plant promoter and the at least one molecule of interest.

In a preferred embodiment of the above methods, the bifactorial endosperm box is introduced into the promoter and into the plant by genetic manipulation techniques.

Preferably, the one or more trans-activating factors are also introduced into the plant by genetic manipulation techniques.

In another preferred embodiment of the above methods, the one or more trans-activating factors are naturally present in the plant.

Even more preferably, the one or more trans-activating factors are overexpressed in the plant.

Preferably, the trans-activating factors bind specifically to one of the G box-like and the prolamine box-like motifs. Even more preferably, the trans-activating factor is an Opaque 2 (O2) bZip protein or a TGA1a-like bZip protein, or both. Where both are present, then preferably they bind competitively with the G box-like motif of the bifactorial endosperm box.

In another preferred embodiment, the trans-activating factor is a PBF protein. More preferably, the trans-activating factor PBF protein binds specifically to the prolamine box-like motif of the bifactorial endosperm box.

In an even more preferred embodiment, the activity of the promoter is further increased by using a trans-activating factor that binds to the prolamine box-like motif of the bifactorial endosperm box and which interacts with one or more of the trans-activating factors that bind to the G box-like motif of the bifactorial endosperm box.

Preferably, the promoter is a plant seed specific promoter, and preferably an endosperm specific promoter. Such promoters are known as such to the skilled person, for example the promoters from the high and low molecular weight wheat glutenins.

Alternatively, the promoter is a plant constitutive promoter. Again, such promoters are known per se by the person skilled in the art, for example, the 35S promoter from cauliflower mosaic virus and the nopaline sythase promoter are two such examples.

In another preferred embodiment of the above method, the at least one molecule of interest is selected from one or more of the groups consisting of therapeutic proteins, immunoglobulins, blood proteins, skin substitutes, collagenic polypeptides, angiomodulators, digestive enzymes, respiratory proteins, anti-cancer molecules, industrial enzymes, diagnostic enzymes, food proteins, herbicidal and insecticidal proteins and plant derived metabolites.

Still yet another object of the present invention is a TGA1a-like transcription or trans-activating factor, obtainable through isolation of nuclear proteins that bind to the consensus nucleic acid sequence "ACTGACGTAAGGGATGACGCAC" or the G-box-like motif of the bifactorial endosperm box described previously.

Another object of the present invention, is a method of regulating the activity of a promoter in a plant, wherein the promoter contains at least one G-box-like motif, and said G-box-like motif binds to one or more TGA1a-like *trans*-activating factors produced in the plant.

In preferred embodiment of this method, the promoter is functionally linked to a nucleic acid sequence coding for a molecule of interest, and the expression of the latter in a recombinant plant host cell is increased through binding of a G-box-like motif present in the promoter to one or more TGA1a-like trans-activating factors produced in the plant.

Yet another object of the present invention is a method of increasing the levels of expression in a specific plant tissue of endogenous or recombinant molecules, wherein the nucleic acid coding sequences of which are under the control of a first plant promoter bearing a G-box-like motif, and a TGA1a like transcription factor is expressed, alone or in combination with another transcription factor, under the control of a second plant promoter that confers expression in the particular tissue.

Still yet another object of the present invention is a method of deregulating the tissue specific activity of a first plant promoter, wherein the promoter contains a G-box-like motif, and a TGA1a like transcription factor is expressed, alone or in combination with another transcription factor, under the control of a second plant promoter that confers expression in all tissues.

Another object of the present invention is a method of modifying the levels of expression of endogenous or recombinant proteins that contain a G-box-like binding site by modifying the structure of a TGA1a like transcription factor, preferably by replacing the activation domain of said TGA1a like transcription factor by an activation domain from another *trans*-activating factor to create a hybrid *trans-*activating factor with greatly increased activating potential.

Yet another object of the present invention is a method of inhibiting the expression in plant host of endogenous or recombinant genes that contain a G-box-like binding site, wherein the TGA1a like transcription factor is expressed in a plant host without its activation domain or its expression is repressed by expression in said plant host of anti-sense RNA complementary to the RNA that encodes for TGA1a like protein.

Thus, one of the major advantages of the discovery of the present applicants is that the bifactorial endosperm box of the present invention can be used to increase the expression of molecules of interest in a recombinant plant cell host, and in particular, pharmaceutical molecules or therapeutic proteins, such as immunoglobulins; blood proteins, e.g. human serum albumin, hemoglobin, thrombin, fibrinogen, and anti-thrombin; hormones for regulating bone, tissue and organ development, and microbicidal or anti-inflammatory function; enzymes used in diagnostics, e.g. b-glucuronidase; skin and interstitial tissue proteins, e.g. collagens; enzymes used in animal feed, e.g. phytase, subtilisin. Another important and surprising advantage is that of being able to make a seed specific plant promoter containing the bifactorial endosperm box of the present invention act like a constitutive promoter through regulation of the bifactorial endosperm box via the binding of transcription factors not normally present in plant tissues other than the endosperm. In this way, it is possible to increase activity of the promoter, and hence any molecule coded by a nucleic acid sequence functionally linked thereto, in substantially all of the tissues of the plant.

The data given in the present application show that *trans*-activating DNA binding factors are able to bind to the bifactorial endosperm box from HMWG-Dx5 promoter and to activate transcription from promoters that contain this specific DNA sequence. Thus, the transcription factors TGA1a like and opaque 2 which are able to bind the G-box-like motif of the bifactorial endosperm box and/or the transcription factor PBF which is able to bind the Pb1 motif of the bifactorial endosperm box, are able to activate and/or deregulate transcription of promoters which contain at least one bifactorial endosperm box and can be used for many purposes:
- increasing the levels of expression in a specific tissue of endogenous or recombinant protein which is under the control of a promoter which contains the bifactorial endosperm box. This can be accomplished by expressing the TGA1a like, or opaque 2, or PBF transcription factors, alone or in combination, under the control of a promoter that confers expression in the particular tissue.
- deregulating the activity of a promoter, which contains the bifactorial endosperm box, and that is specifically expressed in a specific tissue. This can be accomplished by causing the expression of the TGA1a like, or opaque 2, or PBF transcription factors, alone or in combination, under the control of a constitutive promoter that confers expression in all tissues.

The present invention will now be described in more detail with reference to one or more examples of preferred embodiments, in association with the accompanying figures, which examples are non-limiting and serve to illustrate the invention.

### BRIEF DESCRIPTION OF THE FIGURES

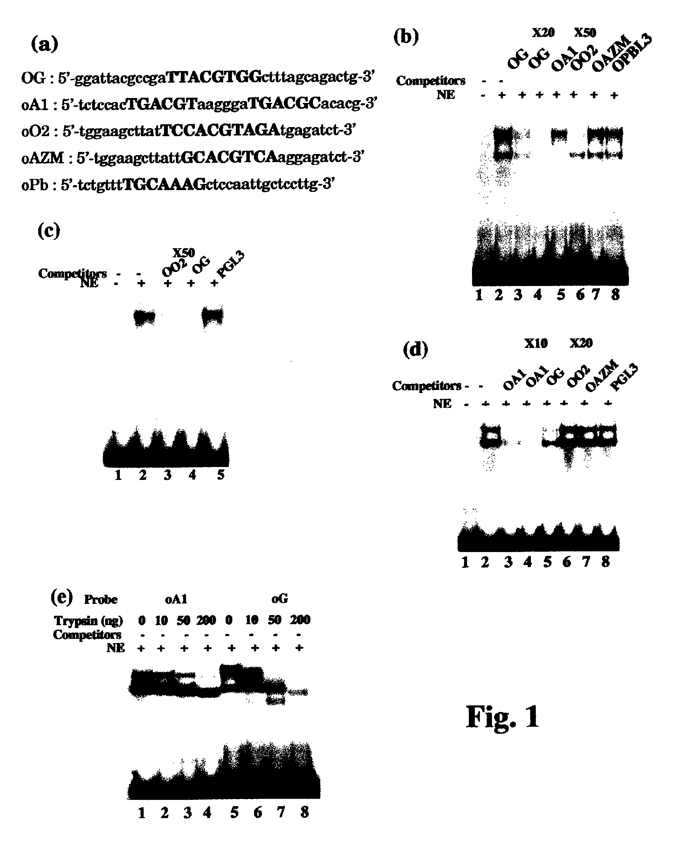

Figures 1(a), 1(b), 1(c), 1(d), and 1(e) represent the characterization and identification of the *in vitro* binding of maize endosperm nuclear proteins to the G-box-like element of the atypical bifactorial endosperm box from HMWG-Dx5 promoter, and in particular:
Figure 1(a) shows sequences of synthetic oligonucleotides containing the as-1 (oA1), G-box-like (oG), Pb1 prolamine box (oPb), Opaque 2 (oO2) and ACGT-like zein (oAZM) motifs (upper cases);
Figure 1(b) shows electrophoretic mobility shift assays of oG specific protein complexes.
Figure 1(c) shows electrophoretic mobility shift assays of oO2 specific protein complexes.
Figure 1(d) shows electrophoretic mobility shift assays of oA1 specific protein complexes.
Figure 1(e) shows electrophoretic mobility shift assays after partial proteolytic clipping of oA1 and oG specific protein complexes.
Figure 2 (a) shows *in vitro* binding of maize endosperm nuclear proteins to the Pb1 element of the atypical bifactorial endosperm box from the HMWG-Dx5 promoter.
Figure 2 (b) shows *in vitro* binding of the wild and mutated purified DNA binding domain of the PBF protein to the Pb1 element of the atypical bifactorial endosperm box from the HMWG-Dx5 promoter.
Figure 3 represents a schematic model of DNA-protein interactions involved in expression of the wheat HMWG-Dx5 promoter in maize endosperm.
Figure 4 represents a schematic drawing of a binary plasmid designated pMRT1222 used for the stable transformation of corn and containing a seed specific promoter in which the bifactorial endosperm box of the present invention is integrated.
Figure 5 represents a schematic drawing of a binary plasmid designated pMRT1477 used for the stable transformation of corn and the endosperm-specific co-expression of the glucuronidase protein as molecule of interest and the PBF protein as a *trans*-activating factor.
Figure 6 represents a schematic drawing of a binary plasmid designated pMRT1512 used for the stable transformation of corn and constitutive co-expression of the *uidA*-IV2 and PBF proteins.

### DETAILED DESCRIPTION OF THE FIGURES

Figure 1(a) shows sequences of synthetic oligonucleotides containing the as-1 (oA1), G-box-like (oG), Pb1 prolamine box (oPb), Opaque 2 (oO2) and ACGT-like zein (oAZM) motifs (upper cases).
Figure 1(b) shows electrophoretic mobility shift assays of oG specific protein complexes. The synthetic oligonucleotide oG was incubated with (lanes 2-8) and without (lane 1) 13 DAP endosperm nuclear extracts (NE). Competition analysis with unlabelled oG (lanes 3-4), oA1 (lane 5), oO2 (lane 6), oAZM (lane 7) and non-specific DNA (lane 8) are shown.
Figure 1(c) shows electrophoretic mobility shift assays of oO2 specific protein complexes. Synthetic oligonucleotide oO2 was incubated with (lanes 2-5) and without (lane 1) 13 DAP endosperm nuclear extracts (NE). Competition analyses with unlabelled oO2 (lanes 3), oG (lane 4), and non-specific DNA (lane 5) are also shown.
Figure 1(d) shows electrophoretic mobility shift assays of oA1 specific protein complexes. The synthetic oligonucleotide oA1 was incubated with (lanes 2-8) and without (lane 1) 13 DAP endosperm nuclear extracts (NE). Competition analysis with unlabelled oA1 (lanes 3-4), oG (lane 5), oO2 (lane 6), oAZM (lane 7) and non-specific DNA (pGL3; lane 8) are shown.
Figure 1(e) shows electrophoretic mobility shift assays after partial proteolytic clipping of oA1 and oG specific protein complexes. The synthetic oligonucleotides oA1 (lanes 1-4) and oG (lanes 5-8) were incubated with endosperm nuclear extracts (NE) and post-incubated without trypsin (lanes 1 and 5) or with 10 ng (lanes 2 and 6), 50 ng (lanes 3 and 7) and 200 ng (lanes 4 and 8) of trypsin.

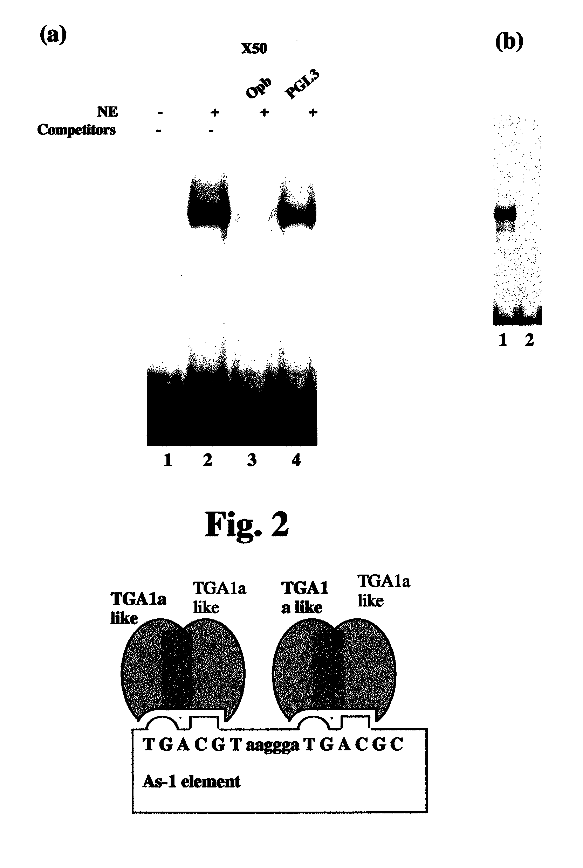

Figure 2 (a) shows *in vitro* binding of maize endosperm nuclear proteins to the Pb1 element of the atypical bifactorial endosperm box from the HMWG-Dx5 promoter. The synthetic oligonucleotide oPb was incubated with (lanes 2-4) and without (lane 1) 13 DAP endosperm nuclear extracts (NE). Competition analysis with unlabelled oPb (lanes 3) and non-specific DNA (lane 4) are shown.
Figure 2 (b) shows i*n vitro* binding of the wild and mutated purified DNA binding domain of the PBF protein to the Pb1 element of the atypical bifactorial endosperm box from the HMWG-Dx5 promoter. The synthetic oligonucleotide oPb was incubated with wild (lanes 1) or mutated (lane 2) purified DNA binding domain of the PBF protein.

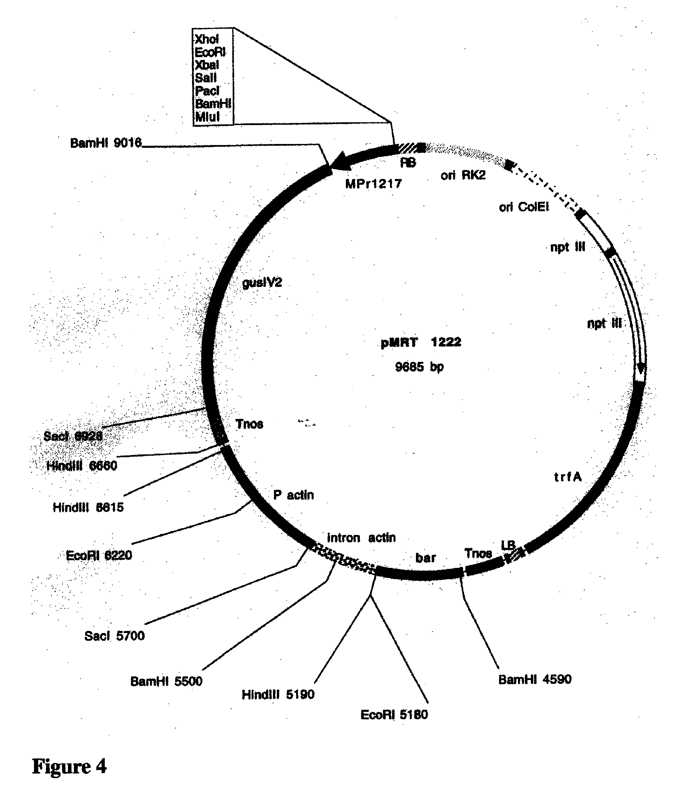

Figure 3 represents a schematic model of DNA-protein interactions involved in expression of the wheat HMWG-Dx5 promoter in maize endosperm. The core sequences of the prolamine box (Pb1) and the G-box-like, which compose the atypical bifactorial endosperm box of HMWG-Dx5 promoter, are shown. The alternative binding of the O2 and TGA1a like bZIP proteins to the G-box-like and the binding of PBF to the Pb1 box are represented schematically. The dashed arrows represent potential interactions of PBF with O2 and TGA1a like motifs. The position of the atypical bifactorial endosperm box relative to transcriptional start site from HMWG-Dx5 promoter is indicated.

### EXAMPLE 1 :

### Molecular characterization of an atypical bifactorial endosperm box from the maize HMWG-Dx5 gene.

### 1 / Materials and methods.

### 1.1 / Maize endosperm nuclear extracts preparation.

Around one hundred 13 DAP maize seeds of the inbred hybrid SN 87 165 (available from Groupe Limagrain) were dissected in order to remove the pericarp, embryo and inner endosperm tissue. After grinding the endosperm in liquid nitrogen using a mortar, the fine powder was homogenized in 20 ml of buffer A (10 mM Hepes, pH 7.8, 10 mM KCL, 10 mM MgCl₂, 5 mM EDTA, 1 mM DTT, 250 mM sucrose, 0.5% Triton X-100 and protease inhibitors) and centrifuged at 4°C for 20 minutes at 3000 *g*. The pellet was washed with 8 ml of buffer A and centrifuged for 15 minutes at 2000 *g*.

The pellet was gently resuspended in low salt buffer (20 mM Hepes, pH 7.8, 20 mM KCL, 1.5 mM MgCl₂, 25% glycerol, 200 µM EDTA, 500 µM DTT and protease inhibitors) in a half volume of the estimated PNV (pellet nuclear volume) and high salt buffer (20 mM Hepes, pH 7.8, 1 M KCL, 1.5 mM MgCl₂, 25% glycerol, 200 µM EDTA, 500 µM DTT and protease inhibitors) was added to reach 35 mS conductivity (350 mM KCl solution was used as standard). The nuclear lysate was incubated for 30 minutes at 4°C with shaking and centrifuged at 12000 g for 20 minutes at 4°C. The supernatant was dialysed against 1000 ml of buffer B (20 mM Hepes, pH 7.8, 100 mM KCL, 10% glycerol, 200 µM EDTA, 500 µM DTT and protease inhibitors) until the conductivity of the extract equalled that of the dialysis buffer (around 1 h). The dialysed extract was centrifuged at 12000 *g* for 30 minutes. Finally, the protein content was quantified by Bradford Assays (BioRad Protein Assay Kit) and aliquots of nuclear extracts were frozen in liquid nitrogen and stored at -80 °C. All procedures were performed at 4°C.

### 1.2 / Gel mobility shift assays.

DNA oligonucleotides used in electrophoretic mobility shift assays (EMSAs) are illustrated in Figure 1a. These sequences are identified in the sequence listing under the numbers SEQ.ID02, SEQ.ID03, SEQ.ID04, SEQ.ID05 and SEQ.ID06 respectively. After annealing of complementary oligonucleotides, double-stranded oligonucleotides were labeled with ³²P-dCTP (3000 Ci mmol⁻¹, Amersham) and Klenow fragment. The labeled DNA probe was purified on MicroSpin™G-25 columns (Pharmacia) and incubated with 3 to 6 µg of endosperm nuclear protein extract in binding buffer [25 mM HEPES, pH7.8, 75 mM KCl, 10% glycerol, 0.5 mM EDTA, 5 mM MgCl₂, 0.45 mM DTT, 1µg Poly(dI-dC).(dI-dC) (Pharmacia) and 25 µg BSA (Biolabs)] for 20 min at room temperature. In competition assays, the endosperm nuclear protein extracts were pre-incubated with the competitor in binding buffer for 20 min on ice. The vector pGL3 (Promega) was used as a non-specific competitor.

DNA-protein complexes were analysed by non-denaturing 5% PAGE in 0.5 x TBE. Following electrophoresis (10 Vcm-1, 3h at 4°C), gels were dried and mounted for autoradiography at -70°C with intensifying screens.

### 1.3 / Enzymatic treatment of protein-DNA complexes.

After binding incubation as described above, the trypsin digestion of protein-DNA complexes was carried out on ice for 15 min as previously described (Skeiky and Iatrou, 1991).

DNA-protein complexes partially proteolyzed were analysed by non-denaturing 5% PAGE in 0.5 x TBE. Following electrophoresis (10 Vcm-1, 3h at 4°C), gels were dried and mounted for autoradiography at -70°C with intensifying screens.

### 2 / Results.

In order to identify nuclear proteins of maize endosperm which selectively bind to the G-box like and the prolamin box (Pb1) elements of the HMWG-Dx5 promoter, electrophoretic mobility shift assays (EMSAs) were performed using the synthetic oligonucleotides illustrated in Figure 1a and identified in the sequence listing under the numbers SEQ.ID02, SEQ.ID03, SEQ.ID04, SEQ.ID05 and SEQ.ID06 respectively.

### 2.1 / At least two maize endosperm proteins bind to the G-box-like element of the atypical bifactorial endosperm box from the HMWG-Dx5 promoter.

As shown in figure 1b three specific oG-protein complexes were obtained using labeled oligonucleotide oG (which bears the G-box-like element from the HMWG-Dx5 promoter) as a probe (lane 2). The faster migrating complex disappeared when unlabeled oligonucleotides oG (lanes 3-4), and oA1, which bears the activation sequence 1 (as-1, Lam et al., 1989) from the Cauliflower mosaic virus (CaMV 35S) promoter, (lane 5) were used, whereas the two slower migrating complexes were competed out using unlabeled oligonucleotides oG (lanes 3-4), and oO2, which bears the "ACGT" core sequence of the Opaque2 (O2) element from 22kDa g-zein promoter, (lane 6). Competition with the unlabeled oAZM, which bears the "ACGT" like core sequence of 27kDa g-zein, (lane 7) and the vector pGL3 (non-specific DNA, lane 8) did not suppress maize endosperm nuclear protein interactions with the G-box-like sequence.

Cross-competition assays demonstrated that the unlabeled oligonucleotide oG was able to compete out complexes obtained with the Opaque 2 element used as a probe (Figure 1c, lane 3). No competition of oO2-protein complexes were observed using the vector pGL3 (Figure 1c, lane 4).

Cross-competition assays also enabled the demonstration that the unlabeled oligonucleotides oG were able to partially compete out the two specific complexes obtained with the as-1 element (Figure 1d), which contains the "TGACG" core sequence repeated in tandem. These two complexes obtained with oA1 oligonucleotide totally disappeared when the amount of unlabeled oG was increased from 20 up to 40 fold molar excess.

No competition of oA1-protein complexes was observed using other unlabeled oligonucleotides such as oO2 (Figure 1d, lane 6), oAZM (Figure 1d, lane 7) and the vector pGL3 (Figure 1d, lane 8).

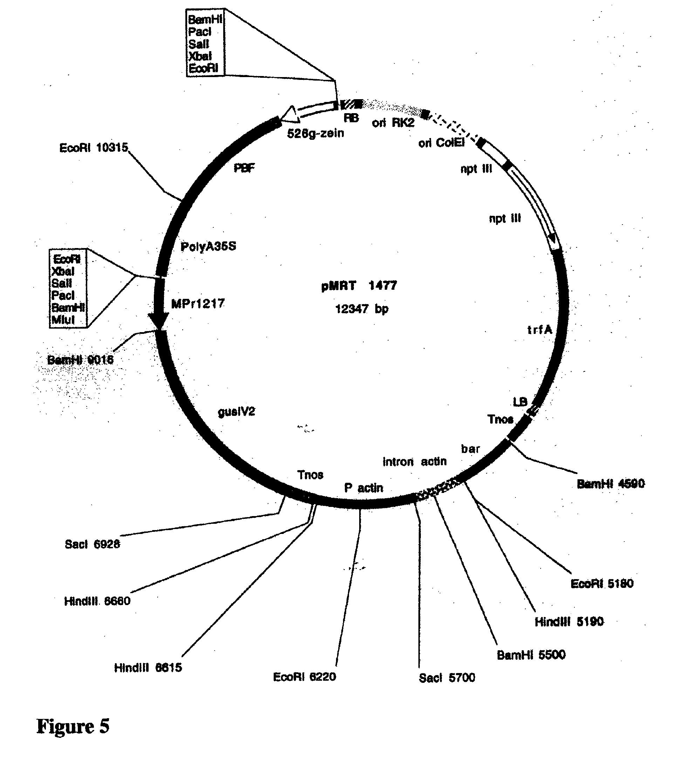

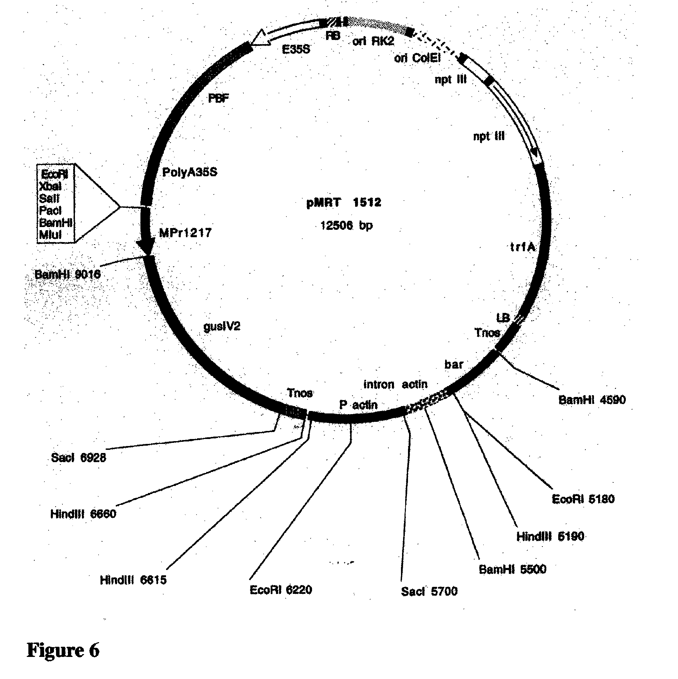

In addition, partial proteolytic clipping assays were carried out. Protein-DNA complexes obtained after incubation of oligonucleotides oG or oA1 with endosperm nuclear protein extracts were partially digested with trypsin. Similar digestion patterns were generated for the two retarded complexes using a oAl probe (Figure 6, lanes 1-4) and for the faster migrating complex using an oG probe (Figure 5e, lanes 5-8). In contrast, the partially proteolyzed patterns generated for the slower migrating complexes using the oG probe were different (Figure 6, lanes 5-8).

### 2.2 / PBF transcription factor is able to bind the Pb1 box of the HMWG-Dx5 promoter.

As shown in figure 2a, a major specific oPb-protein complexe was obtained using labeled oligonucleotide oPb (which bears the Pb1 box from HMWG-Dx5 promoter) as a probe (lane 2). This complex disappeared when unlabeled oligonucleotides oPb1 (lanes 3) were used. Competitions with the vector pGL3 (non-specific DNA, lane 4) did not suppress maize endosperm nuclear protein interactions with the Pb1 sequence.

To confirm that the PBF transcription factor was able to bind the P-box (Pb1) of HMWG-Dx5 promoter we have performed electrophoretic mobility shift assays using wild and mutated purified DNA binding domains of the PBF protein (Figure 2b). A complex was obtained with the wild DNA binding domain (lane 1) while no complex was generated with a mutated DNA binding domain (lane 2) of the PBF transcription factor.

### 3 / Discussion.

The association of a P-box with another regulatory element is known as the bifactorial endosperm box (Hammond-Kosack *et al*., 1993 ; Müller and Knudsen, 1993). This notion is exemplified by the P-box of the 22-kDa g-zein promoter which is followed by an Opaque-2 *cis*-acting motif (Vicente-Carbajosa *et al*., 1997) or by the P-box of 27-kDa zein (Marzábal *et al*., 1998) and LMWG-1D1 (Holdsworth *et al*., 1995) promoters which are followed by a GCN4-like motif. In contrast to reported bifactorial endosperm boxes however, the present invention relates to an atypical bifactorial endosperm box within the HMWG-Dx5 promoter sequence in which the P-box (Pb1) is placed immediately downstream of a G-box-like element.

Gel mobility shift assays indicated that maize endosperm nuclear proteins were able to bind specifically to Pb1 and G-box-like elements of the HMWG-Dx5 bipartite box. It was observed that the PBF endosperm-specific DOF (DNA binding with One Finger) protein of maize, which is known to bind to the prolamin-box of the 22 kDa zein promoter (Vicente-Carbajosa *et al*., 1997), is also able to bind *in vitro* to the 5'-TGCAAAG-3' sequence of Pb1 motif of the HMWG-Dx5 promoter. Several reports have suggested that a cooperative interaction exists between prolamin-box-binding and GCN4-like-binding proteins that appears to be necessary for gene activation of many storage proteins (Wu *et al*., 1998; Albani *et al*., 1997; Vicente-Carbajosa *et al*., 1998). It was demonstrated here that the G-box-like element of HMWG-Dx5 promoter can alternatively bind *in vitro* two *trans*-acting factors. The first identified factor is the endosperm-specific protein bZip Opaque 2 (O2) (Schmidt *et al*., 1990). O2 has been shown to activate genes of storage protein in wheat seeds (Holdworth *et al*., 1995) and rice (Wu *et al*., 1998) through the interaction with GCN4 motifs. The second identified protein which interacts with the G-box-like element in accordance with the method of the present invention belongs to the TGACG binding bZIP factor class (reviewed by Meshi and Iwabuchi, 1995). Indeed, the results presented in the present invention demonstrate that this protein also interacts specifically with both TGACG tandem repeats of the activating sequence 1 (as-1) of CaMV 35S promoter. It has been previously shown that bZip factors binding to the as-1 element as well as related sequences in the promoter of *Arabidopsis glutathione S-transferase 6* can interact with *Arabidopsis* DOF protein OBP1 (Zhang *et al*., 1995; Chen *et al*., 1996), but to the applicants' knowledge, it is the first time that an activating sequence factor-1 (ASF-1) binding affinity is described in endosperm, implying a proximal regulatory sequence of the prolamine storage gene. Without wishing to be bound by theory, it is considered by the present applicants that PBF *via* the Pb1 prolamine-box interacts with O2 and/or TGA1a like transcription factors which can alternatively bind to the G-box-like sequence to activate transcription of HMWG-Dx5 gene in maize endosperm, as demonstrated for example in Figure 3. Furthermore, it was not obvious that the hypothetical model would involve the presence of two proteins which independently interact with the same sequence and seem to have a redundant function, and suggest a competitive interaction of O2 and TGA1a like proteins with the G-box-like motif. It is also believed that if one of the two transcription factors is consumed, then the remaining transcription factor will take over to maintain a high level of activity of the HMWG-Dx5 promoter in maize endosperm.

It is also suggested that in the MPr1217 promoter, published in WO 01/23593, and incorporation of which is made in the present application in relation to said promoter and the way in which it is obtained for the purposes of the present description, and which contains 3 atypical bifactorial endosperm boxes in tandem repeat, that at least two functional *trans*-acting units constituted of the PBF and O2 or PBF and TGA1a like proteins are simultaneously present.

### EXAMPLE 2 :

### Isolation of the TGA1a like transcription factor(s) from maize endosperm that interact with the G-box-like element in the atypical bifactorial endosperm box.

The data shows that there is a *trans*-activating DNA binding factor in maize endosperm that is able to bind to the G-box-like motif of the bifactorial endosperm box from HMWG-Dx5 promoter. This TGA1a-like transcription factor, *via* the G-box-like motif of the bifactorial endosperm box, is able to regulate the transcription of promoters which contain at least one G-box-like motif and can be used for many purposes:
- increasing the levels of expression in a specific tissue of endogenous or recombinant protein which is under the control of a promoter bearing a G-box-like motif. This can be accomplished by causing the expression of the TGA1a like transcription factor, alone or in combination with another transcription factor, under the control of a promoter that confers expression in the particular tissue.
- deregulating the activity of a promoter, which contains a G-box-like motif, and that is specifically expressed in a specific tissue. This can be accomplished by causing the expression of the TGA1a like transcription factor, alone or in combination with another transcription factor, under the control of a constitutive promoter that confers expression in all tissues.
- changing the levels of expression of endogenous or recombinant genes that contain the G-box-like binding site by modifying the structure of the TGA1a like transcription factor. This can be accomplished by replacing the activation domain of the TGA1a like transcription factor by an activation domain from another *trans*-activating factor to create a hybrid *trans*-activating factor with greatly increased activating potential.
- inhibiting the expression of endogenous or recombinant genes that contain the G-box-like binding site. This can be accomplished by expressing the activation domain of the TGA1a like transcription factor without the activation domain or by expressing the RNA that is complementary to the RNA, termed "anti-sens RNA", that encodes for TGA1a like protein.

### 1 / Materials and methods.

cDNA AD library screening was realized using the "MATCHMAKER One-Hybrid System" in accordance with the manufacturer's recommendations (Clontech).

### 1.1 / Maize endosperm cDNA library construction.

Total RNA was prepared from maize seeds 13 days after pollination (DAP) of an inbred hybrid SN 87 165 (available from Groupe Limagrain). Each seed was dissected in order to remove the pericarp, embryo and aleurone cell monolayer. After grinding the endosperm in liquid nitrogen using a mortar, the fine powder was homogenized in 1 ml of iodoacetic acid buffer (100 mM *Tris*-HCl, pH 8.5, 60 mM KCL, 30 mM MgCl₂, 2 mM EDTA, 0.33M sucrose and 1% iodoacetic acid) (Langridge *et al*., 1982) and centrifuged 30 sec. at 5000 *g*. The supernatant was recovered, 1 ml of TriReagent was added and total RNA were then isolated in accordance with the TriReagent method (Sigma).

Poly A(+) RNA was obtained using the "Oligotex™ mRNA Mini Kit" in accordance with the manufacturer's recommendations (Qiagen).

cDNA was synthesized using the "cDNA Synthesis Kit" in accordance with the manufacturer's recommendations (Stratagen).

After size selection, *Eco*RI/*Xho*I insert fragments of the cDNA library were fused to the GAL4 activation domain (AD) in the plasmid pGAD424 (Clontech, Bartel *et al*., 1993). Thus, 10 µg of the plasmid pGAD424 were digested with the restriction enzymes *Eco*RI and *Sal*I during 1 h at 37°C. The released vector fragment was isolated on a 0.8% agarose gel using the "Concert Rapid Gel Extraction System" kit and dephosphorylated with 40 U of calf intestine alkaline phosphatase (New England Biolabs) in buffer 3 (1X) at 37°C during 1 h. Ligation reactions were carried out with 5 µl of the *Eco*RI/*Xho*I insert fragments of each cDNA fractions and 100 ng of the treated vector fragment in a 10 µl final reaction mixture containing T4 DNA ligase buffer (1X) and 400 units of T4 DNA ligase (New England Biolabs), using the "GeneAmp PCR System 9700" thermocycler as followed : one cycle at 10°C for 30 sec. and 200 identical cycles each composed of the following steps: 30 sec. at 30°C, 30 sec. at 10°C and 30 sec. at 30°C. *UltraMAX™ DH5a-FT™ Competent cells* (GIBCOBRL), were transformed with 5 µl of the ligation reaction mixture following the manufacturer's recommendations.

The recombinant plasmid DNA of the clones obtained, which were selected on LB medium supplemented with Ampicilin (50 mg/l), was analyzed by PCR using 5'-CTATTCGATGATGAAGATACCCCACCAAACCCA-3' and 5'-GTGAACTTGCGGGGTTTTTCAGTATCTACGAT-3' primers. 5 x 10⁴ *E. coli* transformants were obtained and only 20% bear an insert of an average size of 1 kb. Recombinant plasmid DNA was prepared from a culture composed of 100 ml LB medium supplemented with Ampicilin (50 mg/l). It was extracted according to the alkaline lysis method.

### 1.2 / Reporter vector construction.

The first reporter vector is a plasmid carrying a *HIS3* reporter gene. It was prepared by cloning *Eco*RI/*Xba*I fragment composed of two tandem copies of as-1 regulatory element into the the *Eco*RI/*Xba*I sites of reporter vector pHISi-1 (Clontech). This *Eco*RI/*Xba*I fragment was obtained by annealing 1µg of each oligonucleotides 5'-aattctccactgacgtaagggatgacgcaagggatgacgtaagggatgacgcacacgt-3' and 5'-ctagacgtgtgcgtcatcccttacgtcatcccttgcgtcatcccttacgtcagtggag-3' (Sigma-Genosys Ltd) in 50 mM NaCl, during 5 min. at 85°C followed by a slow cooling. 100 ng of plasmid pHISi-1 were digested with the restriction enzymes *Eco*RI and *Xba*I for 1 h at 37°C. The vector fragment was then dephosphorylated with 40 U of calf intestine alkaline phosphatase (New England Biolabs) in buffer 3 (1X) at 37°C for 1 h. The ligation reaction was carried out with 20 ng of the "as-1 regulatory element" (*Eco*RI/*Xba*I fragment) and 25 ng of pHISi-1 vector fragment thus treated, in a 10 µl final reaction mixture containing T4 DNA ligase buffer (1X) and of 400 units of T4 DNA ligase (New England Biolabs), using the "GeneAmp PCR System 9700" thermocycler as described above. Competent *Escherichia coli* DH5a bacteria were transformed with the entire ligation reaction mixture. The clones were selected on LB medium supplemented with Ampicilin (50 mg/l). The recombinant plasmid DNA was extracted according to the alkaline lysis method and analyzed with enzymatic digestions. The resulting plasmid was called pMRT1467.

The second reporter vector is a plasmid carrying a *LacZ* reporter gene. It was prepared by cloning *Eco*RI/*Sal*I fragment composed of two tandem copies of as-1 regulatory element into *Eco*RI/*Sal*I sites of reporter vector pLacZi (Clontech). This *Eco*RI/*Sal*I fragment was obtained by annealing 1µg of each oligonucleotides 5'-aattctccactgacgtaagggatgacgcaagggatgacgtaagggatgacgcacacgt-3' and 5'-tcgaacgtgtgcgtcatcccttacgtcatcccttgcgtcatcccttacgtcagtggag-3' (Sigma-Genosys Ltd) in 50 mM NaCl, 5 min. at 85°C followed by a slow cooling.

100 ng of pLacZi plasmid were digested with the restriction enzymes *Eco*RI and *Sal*I for 1 h at 37°C. The vector fragment was then dephosphorylated with 40 U of calf intestine alkaline phosphatase (New England Biolabs) in buffer 3 (1X) at 37°C for 1 h. The ligation reaction was carried out with 20 ng of the "as-1 regulatory element" (*Eco*RI/*Sal*I fragment) and 25 ng of pLacZi vector fragments in a 10 µl final reaction mixture containing T4 DNA ligase buffer (1X) and 400 units of T4 DNA ligase (New England Biolabs), using the "GeneAmp PCR System 9700" thermocycler as described above. Competent *Escherichia coli* DH5a bacteria were transformed with the entire ligation reaction mixture. The clones were selected on LB medium supplemented with Ampicilin (50 mg/l). The recombinant plasmid DNA was extracted according to the alkaline lysis method and analyzed with enzymatic digestions. The resulting plasmid was called pMRT1465.

### 1.3 / Dual reporter yeast strain construction.

Firstly, the *HIS3* reporter gene was integrated into the mutated *his3* locus of YM4271 yeast strain genome (Clontech). Thus, 10 µg of the pMRT1467 construct (target-reporter vector) were linearized with the restriction enzyme *Xho*I. Linearization in the 3' untranslated region immediately following the *HIS3* marker significantly increases the efficiency of homologous recombination at the mutated *his3* locus. Then, 10 µg of the linearized pMRT1467 construct were used to transform the YM4271 yeast strain in accordance with the "Small-scale LiAc Yeast Transformation" procedure (Clontech, Yeast Protocols Handbook, Protocol #PT3024-1, Version #PR03065). The pMRT1467 integration into the mutated *his3* locus of YM4271 was controlled on SD-HIS medium and the yeast clone obtained was called Y6.

Secondly, the *URA3* marker gene was integrated into the mutated *ura3* locus of the Y6 yeast strain genome. Thus, 10 µg of the target-reporter vector pMRT1465 were linearized with the restriction enzyme *Nco*I. Linearization within the *URA3* marker significantly increases the efficiency of homologous recombination at the mutated *ura3* locus. Then, 10 µg of the linearized pMRT1465 construct were used to transform the YM4271 yeast strain in accordance with the "Small-scale LiAc Yeast Transformation" procedure (Clontech, Yeast Protocols Handbook, Protocol #PT3024-1, Version #PR03065). The pMRT1465 integration into the mutated *ura3* locus of Y6 was controlled on SD-HIS-URA medium and the yeast clone obtained was called Y16.

### 1.4 / HIS3 and LacZ background expression of Y16 dual reporter yeast strain.

The *HIS3* background expression of the Y16 yeast strain was analyzed. Thus, a single colony was suspended in 1 ml of TE buffer (1X) and 5 µl of the suspension was plated on SD/-HIS,-URA,+ 0, 15, 30, 45 and 60 mM 3-AT (3-amino-1,2,4-triazole), which is a competitive inhibitor of the yeast *HIS3* protein (His3p). The results indicated that the optimum amount of 3-AT to suppress the *HIS3* background expression of the Y16 yeast strain was 15 mM.

The *LacZ* background expression of Y16 yeast strain was also analyzed using the "b-galactosidase Colony-lift Filter Assay" procedure (Clontech, Yeast Protocols Handbook, Protocol #PT3024-1, Version #PR03065). Results indicated that the Y16 yeast strain lift turns blue after 45 min. of incubation at 30°C.

### 1.5 / cDNA AD library screening.

To screen the maize endosperm cDNA AD library, the dual reporter yeast strain Y16 was transformed with 24 µg of cDNA library using the "Large-Scale Yeast Transformation" procedure (Clontech, MATCHMAKER One-Hybrid System User Manual, Protocol #PT1031-1, Version #PR71132). Positive clones were selected on SD-HIS-URA-LEU+ 15 mM 3-AT medium. To confirm that these clones were candidates for expressing AD/library proteins that bind to as-1 and G-box-like elements, a "b-galactosidase Colony-lift Filter Assay" was performed as described above.

Competent *Escherichia coli* DH5? bacteria were transformed with plasmid DNA isolated from the yeast positive clones (Clontech, Yeast Protocols Handbook, Protocol #PT3024-1, Version #PR03065). The plasmid DNA of the obtained clones, which were selected on LB medium supplemented with Ampicilin (50 mg/l), was extracted according to the alkaline lysis method and analyzed by DNA sequencing.

### EXAMPLE 3 :

### Over-expression and deregulation of synthetic HMWG-Dx5 derived promoters in transformed maize.

### 1 / Materials and methods.

### 1.1 / Construction of the binary plasmids used in the stable transformation of maize.

### 1.1.1 / Construction of the binary plasmid pMRT1222 containing the MPr1217 promoter.

The binary plasmid pMRT1222 was obtained by inserting the expression cassette Mpr1217, and identified as SEQ.ID22 in WO 01/23593, along with *uidA-*IV2 and the*-nos* terminator of pMRT 1217, as identified and published in WO 01/23593 into the *Hpa*I site of the binary plasmid pMRT1195, described in published as WO 01/18192. These two patent applications are incorporated herein by reference insofar as the identification, and description relating to obtaining the incorporated elements are concerned, for the purposes of the description of the present invention.

In order to do this, 7 µg of plasmid pMRT1217 were digested successively with *Eco*RI and *Xmn*I for 1 h at 37°C. The expression cassette was then isolated on a 0.7% agarose gel using the "Concert Rapid Gel Extraction System" kit, and subjected to the action of 12.5 U of the Klenow fragment (New England Biolabs) for 30 min. at 37°C in 50 mM Tris-HCl pH 7.5/50 mM MgCl2 buffer, of 50 mM dithiothreitol (DTT) and of 60 nmol of each of the dNTPs.

In parallel, 5 µg of binary plasmid pMRT1195 were digested with *Hpa*I for 1 h at 37°C. The linearized vector fragment was then dephosphorylated with 40 U of calf intestine alkaline phosphatase (New England Biolabs) in the presence of buffer 3 (1X) at 37°C for 1 h.

The PCR-ligation was carried out with 100 ng of the expression cassette and 10 ng of treated plasmid pMRT1195 using the "GeneAmp PCR System 9700" thermocycler, as described above. Competent *Escherichia coli* DH5? bacteria were transformed with the entire ligation reaction mixture. The clones were selected on LB medium supplemented with kanamycin (50 mg/l). The recombinant plasmid DNA was extracted according to the alkaline lysis method and analysed with enzymatic digestions.

The obtained plasmid was called pMRT1222 and transferred into the LBA4404-pSB1 *Agrobacterium tumefaciens* strain (Komari *et al*., 1996). This strain is derived from the LBA4404 *Agrobacterium tumefaciens* strain subsequent to the integration of the plasmid pSB1, according to the technique described by Holsters *et al*. (1978). The clones were selected on 2YT medium supplemented with rifampicin (50 mg/l), with kanamycin (50 mg/l) and with tetracycline (5 mg/l). The plasmid DNA was extracted according to the alkaline lysis method, which was modified by adding lysozyme (25 mg/ml) to the cell resuspension buffer. The plasmid DNA was analysed with enzymatic digestions. The recombinant *agrobacterium* clone obtained was called A1222.

### 1.1.2 / Construction of the binary plasmid of endosperm-specific co-expression of the uidA-IV2 [expression cassette "Mpr1217 / uidA-IV2 / term-nos"] and PBF [expression cassette "526?-zein / PBF / polyA-35S"] proteins.

Firstly, the *uidA* gene of the 526?-zein construct (Marzábal *et al*., 1998) was carried over from the PBF gene, the nucleic acid sequence of which is available under the Genbank accession number U82230.

The PBF gene was obtained by digesting 10 µg of plasmid pMF6-pbf with *Eco*RI for 1 h at 37°C. The 1225-bp PBF fragment thus released was isolated on a 0.8% agarose gel using the "Concert Rapid Gel Extraction System" kit and was then subjected to the action of 12.5 U of the Klenow fragment (New England Biolabs) for 30 min. at 37°C in the presence of 50 mM Tris-HCl pH 7.5/50 mM MgCl2 buffer, of 50 mM DTT and of 60 nmol of each of the dNTPs.

In parallel, 10 µg of plasmid 526g-zein were digested using *Sac*I and *Bam*HI for 1 h at 37°C. The vector fragment was isolated on a 0.8% agarose gel using the "Concert Rapid Gel Extraction System" kit and then subjected to the action of 6 U of the T4 DNA polymerase (New England Biolabs) for 30 min. at 37°C in the presence of 40 nmol of each of the dNTPs, of T4 DNA polymerase buffer (1X) and of 6µg of BSA. It was dephosphorylated with 40 U of calf intestine alkaline phosphatase (New England Biolabs) in the presence of buffer 3 (1X) at 37°C for 1 h.

The ligation reaction was carried out with 100 ng of the PBF fragment and 10 ng of vector fragment in a 10 µl final reaction mixture containing the T4 DNA ligase buffer (1X) and 400 units of T4 DNA ligase (New England Biolabs), using the "GeneAmp PCR System 9700" thermocycler as described above. Competent *Escherichia coli* DH5? bacteria were transformed with the entire ligation reaction mixture. The clones were selected on LB medium supplemented with ampicillin (50 mg/l). The plasmid DNA was extracted according to the alkaline lysis method and analysed with enzymatic digestions. The resulting plasmid was called pMRT1434.

In a second step, the "526?-zein promoter / PBF gene" from pMRT1434 was subcloned into the binary plasmid pMRT1175 upstream of the polyA35S identified and described in WO 01/18192, incorporated herein by reference in relation to the identification, and description thereof for the purposes of the present application.

In order to do this, 10 µg of plasmid pMRT1434 were digested with the restriction enzyme *Eco*RI for 1 h at 37°C. The released "526?-zein promoter / PBF gene" fragment was isolated on a 0.8% agarose gel using the "Concert Rapid Gel Extraction System" kit.

In parallel, 10 µg of plasmid pMRT1175 were digested with *Eco*RI for 1 h at 37°C and dephosphorylated with 40 U of calf intestine alkaline phosphatase (New England Biolabs) in the presence of buffer 3 (1X) at 37°C for 1 h.

The ligation reaction was carried out with 10 ng of the insert fragment and 30 ng of vector fragment in a 10 µl final reaction mixture in the presence of the T4 DNA ligase buffer (1X) and 400 units of T4 DNA ligase (New England Biolabs), using the "GeneAmp PCR System 9700" thermocycler as described above. Competent *Escherichia coli* DH5? bacteria were transformed with the entire ligation reaction mixture. The clones were selected on LB medium supplemented with Kanamycin (50 mg/l). The plasmid DNA was extracted according to the alkaline lysis method and analyzed with enzymatic digestions. The resulting plasmid was called pMRT1470.

Thirdly, we have cloning the "526?-zein / PBF / polyA35S" cassette in the binary plasmid pMRT1222 which already bears the "MPr1217 / uidAIV2 / term-nos" expression cassette.

The "526?-zein / PBF / polyA35S" fragment was obtained by digesting 10 µg of plasmid pMRT1470 with the restriction enzyme *Mlu*I and *Xho*I for 1 h at 37°C. The released DNA fragment was isolated on a 0.8% agarose gel using the "Concert Rapid Gel Extraction System" kit and was then subjected to the action of 12.5 U of the Klenow fragment (New England Biolabs) for 30 min. at 37°C in the presence of *50 mM Tris-HCl pH 7.5*/*50 mM MgCl2* buffer, of 50 mM DTT and of 60 nmol of each of the dNTPs.

In parallel, 10 µg of plasmid pMRT1222 were digested with *Xho*I for 1 h at 37°C. The vector fragment was subjected to the action of 12.5 U of the Klenow fragment (New England Biolabs) for 30 min. at 37°C in the presence of *50 mM Tris-HCl pH 7.5*/*50 mM MgCl2* buffer, of 50 mM DTT and of 60 nmol of each of the dNTPs, and then dephosphorylated with 40 U of calf intestine alkaline phosphatase (New England Biolabs) in the presence of buffer 3 (1X) at 37°C for 1 h.

The ligation reaction was carried out with 100 ng of the "526g-zein / PBF /polyA35S" fragment and 10 ng of pMRT1222 vector fragment in a 10 µl final reaction mixture in the presence of the T4 DNA ligase buffer (1X) and 400 units of T4 DNA ligase (New England Biolabs), using the "GeneAmp PCR System 9700" thermocycler as described above. Competent *Escherichia coli* DH5? bacteria were transformed with the entire ligation reaction mixture. The clones were selected on LB medium supplemented with Kanamycin (50 mg/l). The plasmid DNA was extracted according to the alkaline lysis method and analyzed with enzymatic digestions. The resulting plasmid was called pMRT1477.

The plasmid pMRT1477 was transferred into the LBA4404-pSB1 *Agrobacterium tumefaciens* strain (Komari *et al*., 1996), according to the technique described by Holsters *et al*. (1978). The clones were selected on 2YT medium supplemented with rifampicin (50 mg/l), with kanamycin (50 mg/l) and with tetracycline (5 mg/l). The plasmid DNA was extracted according to the alkaline lysis method, which was modified by adding lysozyme (25 mg/ml) to the cell resuspension buffer. The plasmid DNA was analysed with enzymatic digestions, and the recombinant *agrobacterium* clone was called A1477.

### 1.1.3 / Construction of the binary plasmid of constitutive co-expression of the uidA-IV2 [expression cassette "MPr1217 (SEQ. ID22) / uidA-IV2 / term-nos"] and PBF [expression cassette "526g-zein / PBF / polyA-35S"] proteins.

Firstly, the PBF gene, the nucleic acid sequence of which is available under the Genbank accession number U82230 was cloned in the binary plasmid pMRT1205, identified and described in WO 01/18192, and incorporated herein by reference in relation to the identification, and description thereof for the purposes of the present description, under the control of enhanced 35S promoter (E35S) and polyA35S terminator.

To do this, 10 µg of plasmid pMF6-pbf were digested with *Bam*HI et *Sal*I for 1 h at 37°C and the released 1225-bp PBF fragment was isolated on a 0.8% agarose gel using the "Concert Rapid Gel Extraction System" kit

In parallel, 10 µg of plasmid pMRT1205 were digested with *Bam*HI et *Sal*I for 1 h at 37°C. The vector fragment was isolated on a 0.8% agarose gel using the "Concert Rapid Gel Extraction System" kit and then dephosphorylated with 40 U of calf intestine alkaline phosphatase (New England Biolabs) in the presence of buffer 3 (1X) at 37°C for 1 h.

The ligation reaction was carried out with 100 ng of the PBF fragment and 10 ng of vector fragment in a 10 µl final reaction mixture in the presence of the T4 DNA ligase buffer (1X) and 400 units of T4 DNA ligase (New England Biolabs), using the "GeneAmp PCR System 9700" thermocycler as described above. Competent *Escherichia coli* DH5? bacteria were transformed with the entire ligation reaction mixture. The clones were selected on LB medium supplemented with ampicillin (50 mg/l). The plasmid DNA was extracted according to the alkaline lysis method and analysed with enzymatic digestions. The resulting plasmid was called pMRT1511.

In a second step, the "E35S / PBF / polyA35S" cassette was cloned into the binary plasmid pMRT1222 which already comprised the "MPr1217 / *uidA*IV2 / term-nos" expression cassette.

In order to do this, 10 µg of the plasmid pMRT1511 was digested with *Kpn*I and *Xho*I for 1 h at 37°C. The released "E35S / PBF / polyA35S" fragment was isolated on a 0.8% agarose gel using the "Concert Rapid Gel Extraction System" kit and was then subjected to the action of 6 U of the T4 DNA polymerase (New England Biolabs) in the presence of 40 nmol of each of the dNTPs, T4 DNA polymerase buffer (1X) and 6µg of BSA, for 30 min. at 37°C.

In parallel, 10 µg of plasmid pMRT1222 were digested with *Xho*I for 1 h at 37°C. The vector fragment was subjected to the action of 12.5 U of the Klenow fragment (New England Biolabs) for 30 min. at 37°C in the presence of *50 mM Tris-HCl pH 7.5*/*50 mM MgCl2* buffer, of 50 mM DTT and of 60 nmol of each of the dNTPs, and then dephosphorylated with 40 U of calf intestine alkaline phosphatase (New England Biolabs) in the presence of buffer 3 (1X) at 37°C for 1 h.

The ligation reaction was carried out with 100 ng of the "E35S / PBF / polyA35S" fragment and 10 ng of pMRT1222 vector fragment in a 10 µl final reaction mixture in the presence of the T4 DNA ligase buffer (1X) and of 400 units of T4 DNA ligase (New England Biolabs), using the "GeneAmp PCR System 9700" thermocycler as described above. Competent *Escherichia coli* DH5? bacteria were transformed with the entire ligation reaction mixture. The clones were selected on LB medium supplemented with Kanamycin (50 mg/l). The plasmid DNA was extracted according to the alkaline lysis method and analyzed with enzymatic digestions. The resulting plasmid was called pMRT1512.

The plasmid pMRT1512 was transferred into the LBA4404-pSB1 *Agrobacterium tumefaciens* strain (Komari *et al*., 1996), according to the technique described by Holsters *et al*. (1978). The clones were selected on 2YT medium supplemented with rifampicin (50 mg/l), with kanamycin (50 mg/l) and with tetracycline (5 mg/l). The plasmid DNA was extracted according to the alkaline lysis method, which was modified by adding lysozyme (25 mg/ml) to the cell resuspension buffer. The plasmid DNA was analysed with enzymatic digestions, and the recombinant *agrobacterium* clone was called A1512.

### 1.2 / Stable transformation of maize using Agrobacterium tumefaciens.

The technique is that described by Ishida *et al*. (1996). Immature embryos 1.0 to 1.2 mm in length (9 to 14 days after pollination) were washed in LS-inf medium, then immersed in the agrobacteria suspension, prepared as described by Ishida et al. (1996), vortexed for 30 sec., and incubated at room temperature for 5 min.

The treated immature embryos were cultivated on LS-AS medium in the dark at 25°C for 3 days, then transferred onto LSD 1.5 medium supplemented with phosphinotricine at 5 mg/l and cefotaxime at 250 mg/l, in the dark at 25°C for 2 weeks and, finally, placed on *LSD* 1.5 medium supplemented with phosphinotricine at 10 mg/l and cefotaxime at 250 mg/l, in the dark at 25°C for 3 weeks. The generated type I calli were isolated, fragmented and transferred onto LSD 1.5 medium supplemented with phosphinotricine at 10 mg/l and cefotaxime at 250 mg/l, in the dark at 25°C for 3 weeks. Then, the type I calli, which had proliferated, were isolated and placed on LSZ medium supplemented with phosphinotricine at 5 mg/l and cefotaxime at 250 mg/l, under a 16 hours light/8 hours darkness photoperiod at 25°C for 2 to 3 weeks. The regenerated plantlets were then transferred onto LSF 1/2 medium under a 16 hours light/8 hours darkness photoperiod at 25°C for 1 to 2 weeks in a growth chamber and further in a phytotron and a greenhouse.

### 1.3 / Measurement of β-glucuronidase activity in maize endosperm and leaves.

To measure the β-glucuronidase activity, samples of transgenic plants were frozen in liquid nitrogen and ground with the aid of a glass rod mounted on a drill. The powder was then resuspended in extraction buffer (25 mM Tris Phosphate, pH 7.8, 2 mM dithiothreitol, 2 mM 1,2-diaminocyclohexane, N,N,N',N'-tetracetic acid, 10% glycerol, 1% Triton X100) in a proportion of 1 ml of buffer per 250 mg of tissue. The mixture was homogenized and then incubated for 1h at 4°C, before being clarified by centrifugation for 5 min at 16060 g.

The GUS activity was measured on 10 µl of clarified crude extract, with the aid of the "GUS-Light chemiluminescent reporter gene assay" detection kit (Tropix Inc., Bedford, USA) according to the manufacturer's recommendations. Measurement of light emission was carried out using a Lumat LB 9507 luminometer (EGG-Berthold, Bad Wildbad, Germany).

The amount of total protein present in the crude extract was measured according to the Bradford technique (1976), using the "Bio-Rad protein assay" reagent (Bio-Rad, Munich, Germany).

### References

**Albani, D., Hammond-Kosack, M.C.U., Smith, C., Conlan, S., Colot, V., Holdsworth, M.J. and Bevan, M.W.** (1997) The wheat transcriptional activator SPA : a seed-specific bZIP protein that recognizes the GCN4-like motif in the bifactorial endosperm box of prolamin genes. *Plant Cell*, **9,** 171-184.
**Chen, W., Chao, G. and Singh, K.B.** (1996) The promoter of a H2O2-inducible, Arabidopsis glutathione S-transferase gene contains closely linked OBF- and OBP1-binding sites. *Plant J*. **10,** 955-966.
**Hammond-Kosack, M.C.U., Holdsworth, M.J. and Bevan, M.W.** (1993) *In vivo* footprinting of a low molecular weight glutenin gene (LMWG-1D1) in wheat endosperm. *EMBO J*. **12**, 545-554.
**Holdsworth, M.J., Munoz-Blanco, J., Hammond-Kosack, M., Colot, V., Schuch, W. and Bevan, M.W.** (1995). The maize transcription factor Opaque-2 activates a wheat glutenin promoter in plant and yeast cells. *Plant Mol. Biol*. **29**, 711-720.
**Ishida, Y., Saito, H., Ohta, S., Hiei, Y., Komari, T. and Kumashiro, T.** (1996) High efficiency transformation of maize (*Zea mays* L.) mediated by *Agrobacterium tumefaciens. Nature Biotechnology*, **14,** 745-750.
**Katagiri, F., Lam, E. and Chua, NH.** (1989) Two tobacco DNA-binding proteins with homology to the nuclear factor CREB. *Nature*, **340,** 727-730.
**Lam, E., Benfey, P.N., Gilmartin, P.M., Fang, R.X. and Chua, N.H.** (1989) Site-specific mutations alter in vitro factor binding and change promoter expression pattern in transgenic plants. *Proc. Natl. Acad. Sci. USA*, **86,** 7890-7894.
**Marzábal, P., Busk, P.K., Ludevid, M.D. and Torrent, M.** (1998) The bifactorial endosperm box of g-zein gene : characterisation and function of the Pb3 and GZM *cis*-acting elements. *Plant J.* **16**, 41-52.
**Meshi, T. and Iwabuchi, M.** (1995) Plant transcription factors. *Plant Cell Physiol.* 36, 1405-1420.
**Müller, M. and Knudsen, S.** (1993) The nitrogen response of a barley C-Hordein promoter is controlled by positive and negative regulation of the GCN4 and endosperm box. *Plant J.* **4,** 343-355.
**Schindler, U., Beckmann, H. and Cashmore, A.R.** (1992) TGA1 and G-Box binding factors: two distinct classes of Arabidopsis Leucine Zipper proteins compete for the G-box-like element TGACGTGG. *The Plant Cell,* **4,** 1309-1319.
**Schmidt, R.J., Burr, F.A., Aukerman, M.J. and Burr, B.** (1990) Maize regulatory gene opaque-2 encodes a protein with a leucine-zipper motif that binds to zein DNA. *Proc. Natl. Acad. Sci. USA*, **87**, 46-50.
**Schmidt, R.J., Ketudat, M., Aukerman, M.J. and Hoschek, G.** (1992) Opaque-2 is a transcriptional activator that recognizes a specific target site in 22-KD zein genes. *Plant Cell,* **4**, 689-700.
**Singh, K.B.** (1998) Transcriptional regulation in plants : the importance of combinatorial control. *Plant Physiol.* **118**, 1111-1120.
**Vicente-Carbajosa, J., Moose, S.P., Parson, R.L. and Schmidt, R.J.** (1997) A maize zinc-finger protein binds the prolamin box in zein gene promoters and interacts with the basic leucine zipper transcriptional activator Opaque-2. *Proc. Natl. Acad. Sci. USA,* **94,** 7685-7690.
**Vicente-Carbajosa, J., Onate, L., Lara, P., Diaz, I. and Carbonero, P.** (1998) Barley BLZ1: a bZIP transcriptional activator that interacts with endosperm-specific gene promoters. *Plant J.* **13,** 629-640.
**Wu, C-Y., Suzuky, A., Washida, H. and Takaiwa, F.** (1998) The GCN4 motif in a rice glutelin gene is essential for endosperm-specific gene expression and is activated by Opaque-2 in transgenic plant rice. *Plant J.* **14,** 673-684.
**Zhang, B., Chen, W., Foley, R.C., Buttner, M. and Singh, KB.** (1995) Interactions between distinct types of DNA binding proteins enhance binding to ocs element promoter sequences. *Plant Cell,* **7**, 2241-2252.

## Claims

1. Bifactorial endosperm box comprising a 5' upstream G-box-like motif and a 3' downstream prolamine box-like motif.

2. Bifactorial endosperm box according to claim 1, wherein the endosperm box originates from a wheat high molecular weight glutenin (HMWG) promoter.

3. Bifactorial endosperm box according to claim 1 or claim 2, wherein the endosperm box originates from the Dx5 HMWG promoter.

4. Bifactorial endosperm box according to any of claims 1 to 3, wherein the bifactorial endosperm box consists of the nucleic acid sequence identified in the sequence listing as SEQ.ID01.

5. Method of regulating the activity of a promoter in a plant, wherein the promoter contains a bifactorial endosperm box according to any one of preceding claims 1 to 4, and said box binds to one or more trans-activating factors produced in the plant.

6. Method of regulating the activity of a promoter in a plant according to claim 5, wherein the bifactorial endosperm box is introduced into the promoter and into the plant by genetic manipulation techniques.

7. Method of regulating the activity of a promoter in a plant according to claim 5, wherein the one or more trans-activating factors are introduced into the plant by genetic manipulation techniques.

8. Method of regulating the activity of a promoter in a plant according to claim 5, wherein the one or more trans-activating factors are naturally present in the plant.

9. Method of regulating the activity of a promoter in a plant according to claim 5, wherein the one or more trans-activating factors are overexpressed in the plant.

10. Method of regulating the activity of a promoter in a plant according to any one of preceding claims 5 to 9, wherein the trans-activating factors bind specifically to one of the G box-like and the prolamine box-like motifs.

11. Method of regulating the activity of a promoter in a plant according to any one of preceding claims 5 to 10, wherein the trans-activating factor is an Opaque 2 (02) bZip protein.

12. Method of regulating the activity of a promoter in a plant according to any one of preceding claims 5 to 10, wherein the trans-activating factor is a TGA1a-like bZip protein.

13. Method according to claim 10, wherein the trans-activating factors are Opaque 2 and TGA1a-like proteins that bind competitively with the G box-like motif of the bifactorial endosperm box.

14. Method of regulating the activity of a promoter in a plant according to any one of preceding claims 5 to 10, wherein the trans-activating factor is a PBF protein.

15. Method according to claim 14, wherein the trans-activating factor PBF protein binds specifically to the prolamine box-like motif of the bifactorial endosperm box.

16. Method according to any one of claims 10 to 15, wherein the activity of the promoter is further regulated by interaction of the trans-activating factor that binds to the prolamine box-like motif of the bifactorial endosperm box with one or more of the trans-activating factors that bind to the G box-like motif of the bifactorial endosperm box.

17. Method according to any one of claims 5 to 16, wherein the promoter is a plant seed specific promoter, and preferably an endosperm specific promoter.

18. Method according to any one of claims 5 to 16, wherein the promoter is a plant constitutive promoter.

19. Method of increasing the expression of at least one molecule of interest in a recombinant plant host cell, comprising introducing into said plant host cell a promoter, comprising the bifactorial endosperm box of any one of claims 1 to 4, upstream of a nucleic acid sequence coding for at least one molecule of interest, wherein the bifactorial endosperm box binds to one or more trans-activating factors produced in the plant, thereby increasing expression of the plant promoter and the at least one molecule of interest.

20. Method according to claim 19, wherein the bifactorial endosperm box is introduced into the promoter and into the plant by genetic manipulation techniques.

21. Method according to claim 19, wherein the one or more trans-activating factors are introduced into the plant by genetic manipulation techniques.

22. Method according to claim 19, wherein the one or more trans-activating factors are naturally present in the plant.

23. Method according to claim 19, wherein the one or more trans-activating factors are overexpressed in the plant.

24. Method according to any one of preceding claims 19 to 23, wherein the trans-activating factors bind specifically to one of the G box-like and the prolamine box-like motifs.

25. Method according to any one of preceding claims 19 to 24, wherein the trans-activating factor is an Opaque 2 (O2) bZip protein.

26. Method according to any one of preceding claims 19 to 24, wherein the trans-activating factor is a TGA1a-like bZip protein.

27. Method according to claim 24, wherein the trans-activating factors areOpaque 2 and TGA1a-like proteins that bind competitively with the G box-like motif of the bifactorial endosperm box.

28. Method according to any one of preceding claims 19 to 23, wherein the trans-activating factor is a PBF protein.

29. Method according to claim 28, wherein the trans-activating factor PBF protein binds specifically to the prolamine box-like motif of the bifactorial endosperm box.

30. Method according to any one of claims 19 to 29, wherein the activity of the promoter is further increased by using a trans-activating factor that binds to the prolamine box-like motif of the bifactorial endosperm box and which interacts with one or more of the trans-activating factors that bind to the G box-like motif of the bifactorial endosperm box.

31. Method according to any one of claims 19 to 30, wherein the promoter is a plant seed specific promoter, and preferably an endosperm specific promoter.

32. Method according to any one of claims 19 to 30, wherein the promoter is a plant constitutive promoter.

33. Method according to any one of the preceding claims 19 to 32, wherein the at least one molecule of interest is selected from one or more of the groups consisting of proteins, immunoglobulins, blood proteins, skin substitutes, collagenic polypeptides, angiomodulators, industrial enzymes, diagnostic enzymes, and plant derived metabolites.

34. TGA1a-like transcription or trans-activating factor, obtainable through isolation of nuclear proteins that bind to the consensus nucleic acid sequence "ACTGACGTAAGGGATGACGCAC" or the G-box-like motif of the bifactorial endosperm box of any one of claims 1 to 4.

35. Method of regulating the activity of a promoter in a plant, wherein the promoter contains at least one G-box-like motif, and said G-box-like motif binds to one or more TGA1a-like *trans*-activating factors produced in the plant.

36. Method according to claim 35, wherein the the promoter is functionally linked to a nucleic acid sequence coding for a molecule of interest, and the expression of the latter in a recombinant plant host cell is increased through binding of a G-box-like motif present in the promoter to one or more TGA1a-like *trans*-activating factors produced in the plant.

37. Method of increasing the levels of expression in a specific plant tissue of endogenous or recombinant molecules, wherein the nucleic acid coding sequences of which are under the control of a first plant promoter bearing a G-box-like motif, and a TGA1a like transcription factor is expressed, alone or in combination with another transcription factor, under the control of a second plant promoter that confers expression in the particular tissue.

38. Method of deregulating the tissue specific activity of a first plant promoter, wherein the promoter contains a G-box-like motif, and a TGA1a like transcription factor is expressed, alone or in combination with another transcription factor, under the control of a second plant promoter that confers expression in all tissues.

39. Method of modifying the levels of expression of endogenous or recombinant proteins that contain a G-box-like binding site by modifying the structure of a TGA1a like transcription factor, preferably by replacing the activation domain of said TGA1a like transcription factor by an activation domain from another *trans*-activating factor to create a hybrid trans-activating factor with greatly increased activating potential.

40. Method of inhibiting the expression in a plant host of endogenous or recombinant genes that contain a G-box-like binding site, wherein the TGA1a like transcription factor is expressed in a plant host without its activation domain or its expression is repressed by expression in said plant host of anti-sense RNA complementary to the RNA that encodes for TGA1a like protein.


SEQUENCE LISTING

<110> Meristem Therapeutics

<120> Bifactorial Endosperm Box, Trans-activating factor
      binding thereto, and Methods of regulating promoter
      activity.

<130> bifact_ep

<140>
<141>

<160> 12

<170> PatentIn Ver. 2.1

<210> 1
<211> 56
<212> DNA
<213> Triticum aestivum

<220>
<221> misc_feature
<222> (8)..(15)
<223> G-box-like motif

<220>
<221> misc_feature
<222> (44)..(50)
<223> Pb1 box

<400> 1
acgccgatta cgtggcttta gcagactgtc caaaaatctg ttttgcaaag ctccaa      56

<210> 2
<211> 33
<212> DNA
<213> Artificial Sequence

<220>
<223> Description of Artificial Sequence:Oligonucleotide

<400> 2
ggattacgcc gattacgtgg ctttagcaga ctg                               33

<210> 3
<211> 30
<212> DNA
<213> Artificial Sequence

<220>
<223> Description of Artificial Sequence:Oligonucleotide

<400> 3
tctccactga cgtaagggat gacgcacacg                                        30


<210> 4
<211> 29
<212> DNA
<213> Artificial Sequence

<220>
<223> Description of Artificial Sequence:Oligonucleotide

<400> 4
tggaagctta ttccacgtag atgagatct                                         29


<210> 5
<211> 29
<212> DNA
<213> Artificial Sequence

<220>
<223> Description of Artificial Sequence:Oligonucleotide

<400> 5
tggaagctta ttgcacgtca aggagatct                                         29


<210> 6
<211> 30
<212> DNA
<213> Artificial Sequence

<220>
<223> Description of Artificial Sequence:Oligonucleotide

<400> 6
tctgttttgc aaagctccaa ttgctccttg                                        30

<210> 7
<211> 33
<212> DNA
<213> Artificial Sequence

<220>
<223> Description of Artificial Sequence:Oligonucleotide

<400> 7
ctattcgatg atgaagatac cccaccaaac cca                                33


<210> 8
<211> 32
<212> DNA
<213> Artificial Sequence

<220>
<223> Description of Artificial Sequence:Oligonucleotide

<400> 8
gtgaacttgc ggggtttttc agtatctacg at                                 32


<210> 9
<211> 58
<212> DNA
<213> Artificial Sequence

<220>
<223> Description of Artificial Sequence:Oligonucleotide

<400> 9
aattctccac tgacgtaagg gatgacgcaa gggatgacgt aagggatgac gcacacgt   58


<210> 10
<211> 58
<212> DNA
<213> Artificial Sequence

<220>
<223> Description of Artificial Sequence:Oligonucleotide

<400> 10
ctagacgtgt gcgtcatccc ttacgtcatc ccttgcgtca tcccttacgt cagtggag   58

<210> 11
<211> 58
<212> DNA
<213> Artificial Sequence

<220>
<223> Description of Artificial Sequence:Oligonucleotide

<400> 11
aattctccac tgacgtaagg gatgacgcaa gggatgacgt aagggatgac gcacacgt   58


<210> 12
<211> 58
<212> DNA
<213> Artificial Sequence

<220>
<223> Description of Artificial Sequence:Oligonucleotide

<400> 12
tcgaacgtgt gcgtcatccc ttacgtcatc ccttgcgtca tcccttacgt cagtggag   58